# EUROPEAN PATENT SPECIFICATION

(11) **EP 3 532 119 B1**
(45) Date of publication and mention of the grant of the patent: **17.03.2021**
(21) Application number: 17792150.9
(22) Date of filing: 26.10.2017
(51) Int. Cl.: A61M 1/10, A61M 1/12

(54) **CARDIAC PUMP**
HERZPUMPE
POMPE CARDIAQUE

(30) Priority: 27.10.2016 GB 201618173
(43) Date of publication of application: 04.09.2019
(73) Proprietor: Calon Cardio-Technology Ltd., West Glamorgan SA2 8PP (GB)
(72) Inventor: MOLTENI, Alessandra, London Greater London NW6 2PA (GB); REDFEARN, Bryony, Swansea SA2 0SF (GB); FOSTER, Graham, Swansea SA2 8BH (GB); HILL, Peter, Cardiff CF11 9EB (GB); WILLIAMS, Robyn, Swansea SA2 0UR (GB); MORIARTY, Christopher, Cardiff CF11 7QB (GB)
(74) Representative: Haseltine Lake Kempner LLP
(86) International application number: PCT/GB2017/053226
(87) International publication number: WO 2018/078370

(56) References cited:
- EP-A1- 3 069 740
- WO-A1-89/07427
- WO-A1-96/18358
- WO-A2-2016/005803

## Description

This disclosure relates to a cardiac pump having an offset inlet, and particularly, but not exclusively, relates to a cardiac pump having improved washing of a bearing assembly.

### Background

Advanced heart failure is a major global health problem resulting in many thousands of deaths each year and those with the disease endure a very poor quality of life. The treatment options for advanced heart failure, for example drug therapy and cardiac resynchronization (pacemakers), have generally proved unsuccessful and the only option remaining for the patients is heart transplantation. Unfortunately, the number of donor hearts available only meets a fraction of the demand, leaving many people untreated.

Ventricular Assist Devices (VAD) have been gaining increased acceptance over the last decade as an alternative therapy to heart transplantation. The use of VADs has shown that, in most cases, once the device has been implanted, the disease progression is halted, the symptoms of heart failure are relieved, and the patient regains a good quality of life.

VADs can be considered as a viable alternative to treat heart failure and offer hope to the many thousands of heart failure patients for whom a donor heart will not be available.

In general terms, it is known to provide a cardiac pump, such as a VAD, that is suitable for implantation into a ventricle of a human heart. The most common type of these implantable pumps is a miniaturised rotary pump, owing to their small size and mechanical simplicity/reliability. Such known devices have two primary components: a cardiac pump housing, which defines a cardiac pump inlet and a cardiac pump outlet; and a cardiac pump rotor, which is housed within the cardiac pump housing, and which is configured to impart energy to the fluid (eg. see EP3069740).

A requirement for the cardiac pump, therefore, is a bearing system that rotatably supports the cardiac pump rotor within the cardiac pump housing. Bearings systems for cardiac pumps, and generally all rotating machines such as pumps and motors, ideally achieve the fundamental function of permitting rotation of the rotor, whilst providing sufficient constraint to the rotor in all other degrees of freedom, i.e. the bearing system must support the rotor axially, radially and in pitch/yaw.

Desirable functions of bearing systems generally may include low rates of wear and low noise and vibration, and in the case of blood pumps, elimination of features that trap blood, or introduce shear stress or heat in the blood.

In known devices, the cardiac pump rotor may be rotatably supported within the housing using one of a number of different types of bearing systems. In general, there are three types of bearing systems that are utilised in cardiac pumps.

Some cardiac pumps use blood-immersed contact bearings, for example a pair of plain bearings, to rigidly support the rotor within the housing. However, for such plain bearing systems it may be difficult to ensure that the rotor is perfectly entrapped within the contact bearings. Moreover, blood-immersed contact bearings of the prior art may be susceptible to proteinaceous and other biological deposition in the bearings, and also in the region proximate to the bearings and on supporting structures around the bearings.

Other cardiac pumps use non-contact hydrodynamic bearing systems, in which the rotor is supported on a thin film of blood. In order to produce the required levels hydrodynamic lift, hydrodynamic bearing systems require small running clearances. As a consequence, blood that passes through those small running clearances may be subjected to high levels of shear stress, which may have a detrimental effect on the cellular components of the blood, for example by causing haemolysis or platelet activation which may further lead to thrombosis.

Cardiac pumps may also employ non-contact magnetic bearing systems, in which the running clearances between the rotor and the housing may be designed such that large gaps can exist in the bearing and therefore shear-related blood damage in the bearing is reduced. However, it is common to use a passive magnetic bearing system in combination with another manner of support in at least one degree-of-freedom, for example active magnetic control, which may significantly increase the size and complexity of the design, and/or hydrodynamic suspension, which may increase the requirements with regard to manufacturing tolerances or introduce blood damage.

A common problem in all cardiac pumps is flow stasis, and cardiac pumps are carefully designed to manage all areas of flow within the pump. One area in particular where flow stasis may occur is in the region of flow that surrounds a bearing of the cardiac device. It is desirable, therefore, to disrupt any areas of flow stasis that may occur in the region of flow that surrounds a bearing during operation of the cardiac pump.

### Statements of Disclosure

According to an aspect of the present disclosure there is provided a cardiac pump comprising: a cardiac pump housing comprising a blood inlet that is offset from a longitudinal axis of the cardiac pump housing. The blood inlet may be configured to impart a non-uniform pressure distribution to the blood that has flowed through the blood inlet into the cardiac pump housing. In particular, the blood that has flowed through the blood inlet into the cardiac pump housing may have a non-uniform pressure distribution in a radial plane of the cardiac pump housing, i.e. relative to the longitudinal axis of the cardiac pump housing.

The blood inlet may be provided in a body portion of the cardiac pump housing. For example, the cardiac pump housing may be a unitary structure that comprises the blood inlet, e.g. after the assembly of the cardiac pump housing. In some cases, it is known to provide a cardiac pump with a separate inflow cannula configured to attach to the cardiac pump housing and divert blood flow into the cardiac pump housing. In such a case, it is understood that the inlet into a free end of the inflow cannula, e.g. the end of the inflow cannula proximal to the cardiac pump in an implanted state, is not regarded as the blood inlet of the cardiac pump housing.

The cardiac pump housing may define a blood flow path between the blood inlet and a blood outlet of the cardiac pump.

The blood inlet may be configured to direct blood in a radial direction, for example in a direction having a radial component relative to the longitudinal axis of the cardiac pump housing. The blood inlet may be configured to direct blood away from and/or towards the longitudinal axis of the cardiac pump housing. The blood inlet may be configured to establish a cross-flow of blood around, across and/or through another feature of the cardiac pump. For example, the blood inlet may be configured to flow blood in a crosswise manner, e.g. diagonally or transversely, around, across and/or through the cardiac pump housing. The blood inlet may be configured to establish a counterflow against and/or across the flow of blood through the cardiac pump.

In the context of the present disclosure, the term "cardiac pump" is understood to mean any type of pump that is configured to pump blood. For example, the cardiac pump may be a continuous flow pump having a radial, axial or mixed flow regime. The cardiac pump may be a rotary pump.

The cardiac pump may comprise at least one bearing assembly configured to rotatably support a cardiac pump rotor within the cardiac pump housing, thereby defining a rotational axis of the cardiac pump rotor. The blood inlet may be configured to divert radially the flow of blood across the at least one bearing assembly, i.e. towards and/or away from the rotational axis of the cardiac pump.

The at least one bearing assembly may be positioned concentrically with the longitudinal axis of the cardiac pump housing. The longitudinal axis of the cardiac pump housing may be collinear with the rotational axis of the cardiac pump rotor. The longitudinal axis of the cardiac pump housing may be offset from the rotational axis of the cardiac pump rotor. The at least one bearing assembly, for example the centre of rotation of the at least one bearing assembly, may be offset from the rotational axis of the cardiac pump rotor.

The at least one bearing assembly may comprise a contact bearing having a first contact bearing portion configured to engage a second contact bearing portion thereby defining a contact bearing interface. The blood inlet may be configured to divert, e.g. radially divert, the flow of blood across, through, onto and/or around the contact bearing interface of the at least one bearing assembly. The contact bearing interface of the at least one bearing assembly may be positioned downstream of the blood inlet, e.g. at a position that is longitudinally offset from at least a portion of the blood inlet. For example, the cardiac pump may be configured so that blood flows into the cardiac pump housing through the blood inlet and directly onto, across and/or around the contact bearing interface of the at least one bearing assembly, e.g. for the purpose of disrupting any areas of flow stasis that may exist proximate to the contact bearing interface.

Where the cardiac pump comprises the at least one bearing assembly, the position of the blood inlet relative to the at least one bearing assembly, e.g. the contact bearing interface of the at least one bearing assembly, may be a key feature of the cardiac pump.

For example, the relative positions of the blood inlet and the contact bearing interface may be specially selected to ensure that once blood has entered the cardiac pump housing, it continues to flow towards the at least one bearing assembly without diverting away from an outlet of the cardiac pump housing. In other words, the contact bearing interface may be provided a point in the flow path of the blood to ensure that blood entering the cardiac pump housing impinges directly onto the contact bearing interface. The contact bearing interface maybe provided at a location of the cardiac pump housing where it is not shrouded by one or more other features of the cardiac pump housing. Specifically, the blood inlet, e.g. at least one opening provided by the blood inlet into the blood flow path, may be disposed upstream, e.g. axially and/or radially upstream, of the contact bearing interface of the at least one bearing assembly. In the context of the present disclosure, the term "upstream" is understood to mean a point along the flow path of the blood that is located more towards the inlet of the cardiac pump than the outlet of the cardiac pump. Thus, the term "axially upstream" is understood to mean a point along the flow path of the blood that is located more towards the inlet of the cardiac pump than the outlet of the cardiac pump in a direction along the longitudinal axis of the cardiac pump, and the term "radially upstream" is understood to mean a point along the flow path of the blood that is located more towards the inlet of the cardiac pump than the outlet of the cardiac pump in a direction perpendicular to the longitudinal axis of the cardiac pump.

The cardiac pump housing may be configured to extend at least partially through a wall of a heart. For example, the cardiac pump housing may comprise an inlet tube, such as an inflow cannula, configured to extend at least partially through a wall of the heart. The inlet tube may comprise the blood inlet. The inlet tube may be integral, for example unitary, with the cardiac pump housing.

The blood inlet may be positioned within the heart when the cardiac pump is implanted at least partially in the heart. For example, the blood inlet may be provided towards one end of the inlet tube so that the blood inlet is entirely within a portion of the heart, in an implanted state. The cardiac pump may be configured to be implanted entirely within the heart. The cardiac pump may be configured to be implanted entirely outside of the heart.

The blood inlet may define the passage of blood from within the heart to a location within the cardiac pump housing, for example a position within the inlet tube. The blood inlet may comprise a passageway, for example a duct, that extends through a wall of the cardiac pump housing. The passageway may be non-uniform in cross section, for example the cross section of the passageway may vary along the longitudinal axis of the cardiac pump housing.

The passageway may comprise an inner wall configured to direct blood flow radially across cardiac pump housing. The inner wall of the passageway may comprise at least one projection configured to narrow the passageway for blood flow into the cardiac pump housing.

The blood inlet may comprise one or more openings. For example, the blood inlet may comprise a first opening extending through a wall of the cardiac pump housing, and at least one other opening extending through a wall of the cardiac pump housing. The first opening and the at least one other opening may extend in different directions to one another. The first opening and the at least one other opening may intersect one another. The opening may be defined by the minimum cross sectional area of the blood inlet in a radial plane of the cardiac pump housing. For example, the opening may be defined by the narrowest region of a passageway into the cardiac pump housing. The opening may be defined by a portion of the passageway that has a locally reduced cross sectional area.

The one or more openings may have a centre of area that is offset from the longitudinal axis of the cardiac pump housing. For example, where the blood inlet comprises a single opening, the centre of area, e.g. the centroid of the opening in a radial plane of the cardiac pump housing, may be offset from the longitudinal axis of the cardiac pump housing. Where the blood inlet comprises a plurality of openings, the overall centre of area, i.e. the combined centre of areas of the plurality of openings, may be offset from the longitudinal axis of the cardiac pump housing. In this manner, the net flow of the blood into the cardiac pump housing may be radially offset from the longitudinal axis of the cardiac pump housing.

Where the blood inlet comprises a single opening, the mean axial component of the blood flow through the single opening may be radially offset from the longitudinal axis of the cardiac pump housing. Where the blood inlet comprises a plurality of openings, the mean axial components of the blood flow through each of the openings may be summed so that the overall axial component of the total blood flow through the blood inlet is radially offset from the longitudinal axis of the cardiac pump housing.

The blood inlet may be positioned asymmetrically in a radial plane of the cardiac pump housing. The blood inlet may have a rotational symmetry of order 1, i.e. no rotational symmetry, about the longitudinal axis of the cardiac pump housing. The blood inlet may be non-axisymmetric, for example about the longitudinal axis of the cardiac pump housing. Each opening of the blood inlet may be non-axisymmetric, for example about an axis extending through the centre of area of the opening.

Each of the openings may be any appropriate shape. For example, the blood inlet may comprise one or more openings having a circular, elliptical, oval, crescent, triangular, square or rectangular shape, or any other appropriate shape. In particular, the cross-sectional profile of each opening, for example the cross-sectional profile in a plane perpendicular to the mean flow path of blood through the opening, may have a circular, elliptical, oval, crescent, triangular, square or rectangular shape, or any other appropriate shape.

Each opening may act to constrict the flow of blood into the cardiac pump housing. The blood inlet may comprise a nozzle configured to restrict the flow of blood. Each opening may be configured to accelerate the flow of blood into the cardiac pump housing. The blood inlet may comprise at least one projection that extends into the flow of blood through the inlet. The at least one projection may be configured to disturb the pressure distribution in the blood inlet, for example to cause a non-uniform pressure distribution in a radial plane of the cardiac pump housing. The at least one projection may be configured to impart a non-uniform pressure distribution to the blood. The at least one projection may be configured to divert the flow of blood, for example divert the flow of blood towards another feature of the cardiac pump, such as a bearing assembly.

The cardiac pump may comprise a primary flow path, which is defined as the flow of blood between the blood inlet and the blood outlet of the cardiac pump. The cardiac pump may further comprise a secondary flow path, which is defined as any recirculating flow inside the cardiac pump that does not form part of the primary flow path. For example, the cardiac pump may comprise one or more regions of secondary flow around the bearing assembly. The blood inlet may be configured to disrupt areas of flow stasis, for example areas of flow stasis in the primary and/or secondary flow. The blood inlet may be configured to establish a counterflow across the primary flow path of blood through the cardiac pump.

The cross sectional area of the flow through the blood inlet may be larger than, approximately the same size as or smaller than the cross sectional area of a region of flow in the cardiac pump housing, for example a region of flow proximate to the blood inlet. The bearing assembly may be positioned in the region of flow proximate to the blood inlet. The region of flow proximate to the blood inlet may comprise a portion of primary flow and/or a portion of secondary flow. The region of flow proximate to the blood inlet may have a cross sectional area in a radial plane of the cardiac pump housing that is coincident with the contact bearing interface.

The ratio of the cross sectional area of the region of flow proximate to the blood inlet to the cross sectional area of the flow through the blood inlet may be in the range of approximately 1:0.2 to 1:1. The ratio of the cross sectional area of the region of flow proximate to the blood inlet to the cross sectional area of the flow through the blood inlet may be in the range of approximately 1:0.4 to 1:1. The ratio of the cross sectional area of the region of flow proximate to the blood inlet to the cross sectional area of the flow through the blood inlet may be in the range of approximately 1:0.4 to 1:0.9. The ratio of the cross sectional area of the region of flow proximate to the blood inlet to the cross sectional area of the flow through the blood inlet may be in the range of approximately 1:0.4 to 1:0.65. In this manner, the flow of blood through the blood inlet may be at least the same as, or faster, than the flow of blood in the region of flow proximate to the blood inlet. Example locations of various cross sections through the cardiac pump are described in the below description and shown in the appended figures.

For example, where the blood inlet comprises a single opening, the cross sectional area of the region of flow proximate to the blood inlet may be approximately the same size as, or larger than the cross sectional area of the flow through the single opening of the blood inlet. Where the blood inlet comprises a plurality of openings, the cross sectional area of the region of flow proximate to the blood inlet may be approximately the same size as, or larger than the total cross sectional area of the flow through the plurality of openings of the blood inlet.

The offset blood inlet may be configured to flow blood from outside of the cardiac pump housing to the inside of the cardiac pump housing such that blood washes onto, around, through and/or across the contact interface of the bearing assembly. The present disclosure is advantageous since the blood inlet is offset from the longitudinal axis of the blood inlet, which causes the blood flow to have a significant radial flow component in the region surrounding the bearing assembly. As a result, the present disclosure serves to mitigate the formation of areas of flow stasis that may be associated with deposition of proteins and/or thrombus formation in those regions of flow surrounding the at least one bearing assembly.

The cardiac pump may comprise an annular blood gap between the cardiac pump housing and the cardiac pump rotor when the cardiac pump is in an assembled configuration. The blood inlet may be configured to flow blood into the annular blood gap. The bearing assembly may be positioned in between the blood inlet and the annular blood gap.

The cross sectional area of the annular blood gap may be smaller than, approximately the same size as, or larger than the cross sectional area of the flow through the blood inlet. For example, where the blood inlet comprises a single opening, the cross sectional area of the annular blood gap may be smaller than, approximately the same size as, or larger than the cross sectional area of the flow through the single opening of the blood inlet. Where the blood inlet comprises a plurality of openings, the cross sectional area of the annular blood gap may be smaller than, approximately the same size as, or larger than the total cross sectional area of the flow through the plurality of openings of the blood inlet.

The ratio of the cross sectional area of the annular blood gap to the cross sectional area of the flow through the blood inlet may be in the range of approximately 1:0.2 to 1:3. The ratio of the cross sectional area of the annular blood gap to the cross sectional area of the flow through the blood inlet may be range of approximately 1:0.8 to 1:2.5. The ratio of the cross sectional area of the annular blood gap to the cross sectional area of the flow through the blood inlet may be range of approximately 1:0.8 to 1:1.9. The ratio of the cross sectional area of the annular blood gap to the cross sectional area of the flow through the blood inlet may be range of approximately 1:0.8 to 1:1.45. In this manner, the flow of blood through the blood inlet may be slower than, approximately the same as, or faster than the flow of blood through the annular flow gap, or indeed the average flow of the primary flow of blood through the cardiac pump.

The cross sectional area of the flow through the blood inlet may be selected depending on the flow characteristics of the blood in the cardiac pump housing. For example, the cross sectional area of the flow through the blood inlet may be determined as a function of the cross sectional area of the region of flow proximate to the blood inlet and/or the cross sectional area of the annular blood gap.

According to another aspect of the present disclosure there is provided a cardiac pump housing comprising a blood inlet is configured to cause a non-uniform pressure distribution in a radial plane of the cardiac pump housing.

According to another aspect of the present disclosure there is provided a cardiac pump comprising one or more features configured to cause a non-uniform pressure distribution in a radial plane of the cardiac pump housing.

According to another aspect of the present disclosure there is provided a cardiac pump comprising one or more features configured to disturb a flow regime established by operation of the cardiac pump, for example a flow regime established by the pumping work of an impeller of the cardiac pump. For example, the cardiac pump may comprise one or more projections that extend into the blood flow, the projections being configured to disturb the flow regime of the blood to cause a non-uniform pressure distribution in the region surrounding the projection. The projection may be provided in a region of flow close to a bearing assembly of the cardiac pump, so that the projection causes a non-uniform pressure distribution in the blood flow surrounding the bearing assembly.

According to another aspect of the present disclosure there is provided a cardiac pump comprising: a cardiac pump housing comprising an inlet tube configured to extend at least partially through a wall of a heart, the inlet tube comprising a blood inlet positioned within the heart when the cardiac pump is implanted in the heart, wherein the blood inlet is configured to cause a non-uniform pressure distribution in a radial plane of the inlet tube.

To avoid unnecessary duplication of effort and repetition of text in the specification, certain features are described in relation to only one or several aspects or arrangements of the disclosure. However, it is to be understood that, where it is technically possible, features described in relation to any aspect or arrangement of the disclosure may also be used with any other aspect or arrangement of the disclosure.

### Brief Description of the Drawings

For a better understanding of the present disclosure, and to show more clearly how it may be carried into effect, reference will now be made, by way of example, to the accompanying drawings, in which:
Figure 1 shows a cut-away of a heart with a cardiac pump implanted into the left ventricle;
Figure 2 shows a perspective view of a cardiac pump in an assembled configuration;
Figure 3 shows a cross sectional view of the cardiac pump of figure 2 in an assembled configuration;
Figure 4 shows a partial cross sectional view of the cardiac pump of figures 2 and 3;
Figure 5 shows a partial cross sectional view of a cardiac pump;
Figure 6 shows a cross sectional view of another cardiac pump in an assembled configuration;
Figure 7 shows a partial cross sectional view of the cardiac pump of figure 6;
Figure 8 shows a partial cross sectional view of another cardiac pump;
Figure 9 shows a partial cross sectional view of another cardiac pump;
Figure 10 shows a perspective view of another cardiac pump in an assembled configuration; and
Figure 11 shows a cross sectional view of the cardiac pump of figure 10 in an assembled configuration.

### Detailed Description

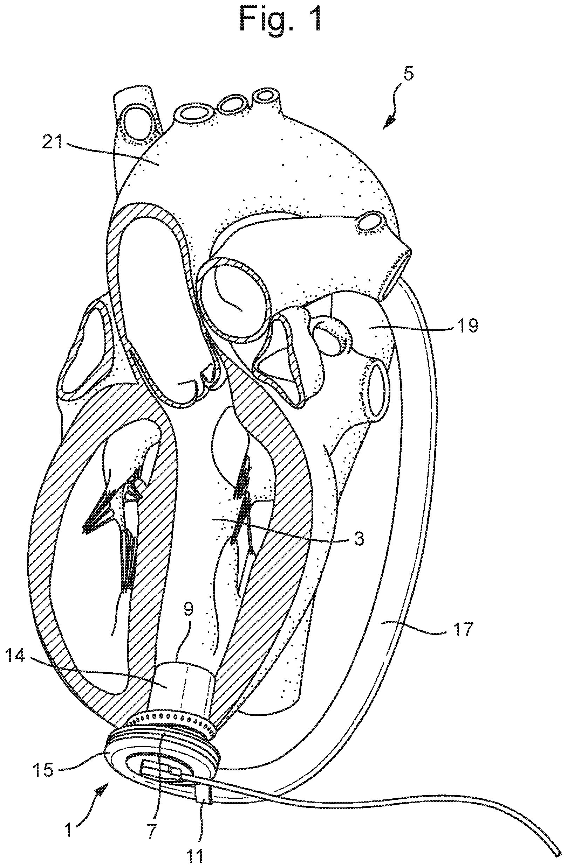

Figure 1 depicts a cardiac pump 1 for the treatment of heart failure, for example a Ventricular Assist Device (VAD), in an implanted configuration in the left ventricle 3 of a heart 5. The cardiac pump 1 according to the present disclosure may be any appropriate type of cardiac pump. For example, the cardiac pump 1 may be an axial flow cardiac pump, a radial flow cardiac pump, or a mixed flow cardiac pump. It is understood, therefore, that where technically possible, features described in relation to a radial flow cardiac pump may be employed in any type of cardiac pump, such as an axial flow cardiac pump. Further, whilst figure 1 depicts the cardiac pump in an implanted configuration in the left ventricle 3 of a heart 5, it is understood that the cardiac pump 1 may be implanted in any appropriate position, for example completely outside of the heart 5 or completely inside of the heart 5.

The cardiac pump 1 of figure 1 comprises a cardiac pump housing 7 comprising an inlet 9 for blood and an outlet 11 for blood. The cardiac pump 1 comprises a cardiac pump rotor disposed at least partially within the cardiac pump housing 7. The cardiac pump rotor is supported, for example rotatably supported, by way of one or more bearing assemblies, as described below.

The cardiac pump 1 comprises an inflow tube, for example an inflow cannula 14, that is integral to the cardiac pump housing 7. When the cardiac pump is in an implanted state, the inflow cannula 14 is situated at least partially inside the left ventricle 3, with a pumping chamber 15 being situated outside of the heart 5. The inflow cannula 14 extends between the pumping chamber 15, through the wall of the left ventricle 3 into the chamber of the left ventricle 3, so that the inlet 9 is situated completely within the left ventricle 3. The pumping chamber 15 is situated on the apex of the left ventricle 3 with the outlet 11 connected to a separate outflow cannula 17. In the example shown in figure 1, the outflow cannula 17 is anastomosed to a descending aorta 19, although in an alternative example the outflow cannula 17 may be anastomosed to an ascending aorta 21.

Although not shown in any of the figures, the cardiac pump 1 may comprise a magnetic drive coupling, for example a brushless DC motor. The cardiac pump rotor 8 may comprise a first portion of the magnetic drive coupling, for example one or more permanent magnets. The cardiac pump housing 7 may comprise a second portion of the magnetic drive coupling, for example one or more electrical windings. The magnetic drive coupling may be a radial magnetic drive coupling, e.g. a radial flux gap electric motor, although it is appreciated that the magnetic drive coupling may be of any appropriate configuration.

One of the most important factors in the design of a VAD is the passage of blood through the cardiac pump 1, particularly the passage of blood in the region of the bearings. The regions of blood flow around the bearings, i.e. the regions around the circumferential transition between the rotating and stationary components of a plain bearing assembly, may be areas of flow stasis and therefore predisposed to thrombus formation or indeed any type of protein deposition. It is particularly important, therefore, that bearings are well washed with a constant supply of fresh blood as the heat generated and geometrical constraints in these areas make them particularly prone to thrombus formation and/or pump deposition.

Therefore, it is desirable to directly expose one or more surfaces of the bearing, for example the interface between rotating and stationary components of the bearing, to a continuous supply of blood flow, such that the proteinaceous and cellular components of the blood responsible for pump deposition and thrombus formation are prevented from aggregating in this region.

The present disclosure relates to a cardiac pump 1 that reduces the risk of damage to the cellular components of the blood. For example, the cardiac pump 1 according to the present disclosure may mitigate the deposition of proteins and/or the formation of thrombi within the cardiac pump 1, and in particular, may mitigate the deposition of proteins and/or the formation of thrombi in areas proximate to one or more bearing assemblies of the cardiac pump 1.

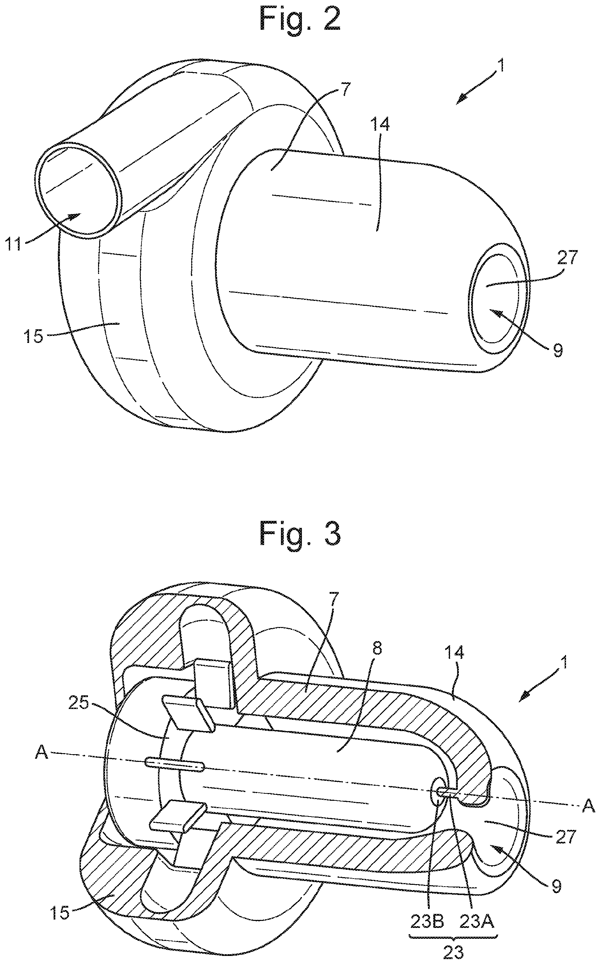

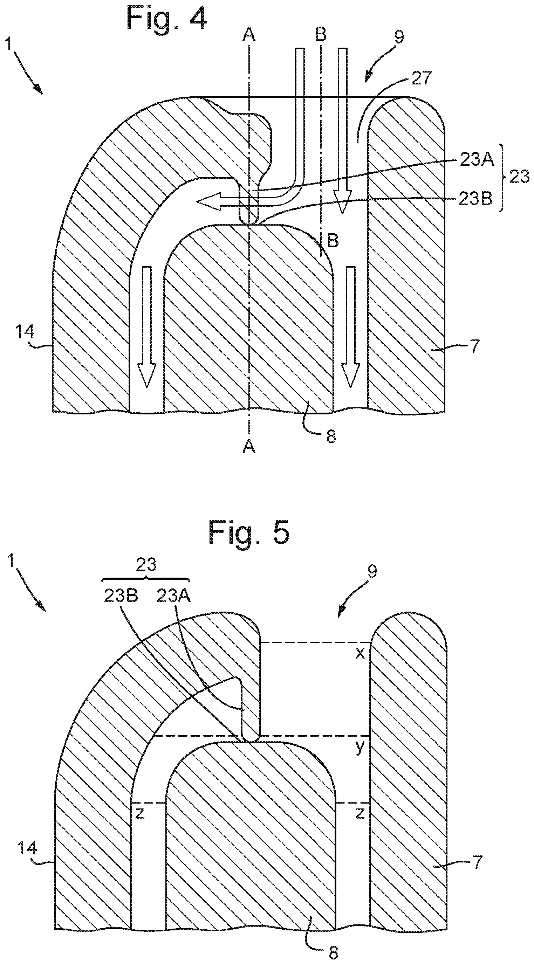

Figure 2 shows one arrangement of the cardiac pump 1 and figure 3 shows a cross-section through the cardiac pump 1 along a longitudinal axis A-A. Figure 4 shows a partial cross-section through the cardiac pump 1 along a longitudinal axis A-A, which shows the flow of blood though the inlet 9 of the cardiac pump 1.

The cardiac pump housing 7 is configured to rotatably support a cardiac pump rotor 8 at least partially within the cardiac pump housing 7. The cardiac pump rotor 8 is rotatably coupled to an impeller portion 25 which is configured to pump the blood and which may be provided at or towards an end of the cardiac pump rotor 8. The cardiac pump rotor 8 may be supported by one or more types of appropriate bearing assemblies, such that the cardiac pump rotor 8 is substantially constrained, e.g. in five degrees-of-freedom, and the cardiac pump rotor 8 may rotate about the longitudinal axis A-A. In other words, a bearing system of the cardiac pump 1 permits rotation of the cardiac pump rotor 8, which is a fundamental function of the bearing system, and provides sufficient constraint to the cardiac pump rotor 8 in all other degrees of freedom. In this manner, the bearing system supports the cardiac pump rotor 8 in the axial and radial directions, as well as in pitch and yaw. For example, the cardiac pump rotor 8 may be supported by a first plain bearing assembly and a second plain bearing assembly. Additionally or alternatively, the cardiac pump 1 may comprise one or more magnetic bearing assemblies and/or one or more electromagnetic bearing assemblies. In the example shown in figures 2 to 11, the cardiac pump rotor 8 is partially supported by a plain bearing assembly 23 located towards the inlet 9 of the cardiac pump, and it is understood that the plain bearing assembly 23 supports the cardiac pump rotor 8 in combination with at least one other bearing assembly. It is appreciated, however, that any of the bearing assemblies of the cardiac pump 1 may be positioned at any appropriate portion of the cardiac pump 1, dependent upon the operational requirements of the cardiac pump 1.

The plain bearing assembly 23 is a type of contact bearing assembly in which the bearing surfaces of the plain bearing assembly 23 are configured to be in contact during operation of the cardiac pump 1. For example, the plain bearing assembly 23 may comprise no intermediate rolling elements, i.e. motion is transmitted directly between two or more contacted surfaces of respective portions of the plain bearing assembly 23.

The plain bearing assembly 23 comprises a first plain bearing portion 23a. The first plain bearing portion 23a is coupled to the cardiac pump rotor 8 such that, during operation of the cardiac pump 1, the first plain bearing portion 23a does not rotate with the cardiac pump rotor 8. In the examples shown in figures 3 to 9 and 11, the first plain bearing portion 23a is integral to the cardiac pump housing 7, although in an alternative example (not shown) the first plain bearing portion 23a may be a separate component rigidly fixed to the cardiac pump housing 7. In another example, the first plain bearing portion 23a may be movably coupled, for example threadably coupled, to the cardiac pump housing 7 such that the position of the first plain bearing portion 23a may be adjusted relative to the cardiac pump housing 7. The first plain bearing portion 23a may be constructed from a different material to the cardiac pump housing 7, e.g. a ceramic material. Alternatively, the first plain bearing portion 23a may be constructed from a similar material to the cardiac pump housing 7, e.g. a titanium alloy. The first plain bearing portion 23a may comprise a surface coating and/or may have had a surface treatment to improve the wear characteristics of the plain bearing assembly 23.

The plain bearing assembly 23 comprises a second plain bearing portion 23b. The second plain bearing portion 23b is coupled to the cardiac pump rotor 8 such that, during operation of the cardiac pump 1, the second plain bearing portion 23b rotates with the cardiac pump rotor 8. In the example shown in figures 3 and 4, the second plain bearing portion 23b is integral to the cardiac pump rotor 8, although in an alternative example the second plain bearing portion 23b may be a separate component rigidly fixed to the cardiac pump rotor 8. In another example, the second plain bearing portion 23b may be movably coupled, for example threadably coupled, to the cardiac pump rotor 8 such that the position of the second plain bearing portion 23b may be adjusted relative to the cardiac pump rotor 8. The second plain bearing portion 23b may be constructed from a different material to the cardiac pump rotor 8, e.g. a ceramic material. Alternatively, the second plain bearing portion 23b may be constructed from a similar material to the cardiac pump rotor 8, e.g. a titanium alloy. The second plain bearing portion 23b may comprise a surface coating and/or may have had a surface treatment to improve the wear characteristics of the plain bearing assembly 23. The first and second plain bearing portions 23a, 23b may be constructed from different materials to each other, for example the first and second plain bearing portions 23a, 23b may each be constructed from a different ceramic material.

The first and second plain bearing portions 23a, 23b are configured to engage each other so as to be in contact when the cardiac pump rotor 8 and the cardiac pump housing 7 are in an assembled configuration, such that the plain bearing assembly 23 is configured to rotatably support the cardiac pump rotor 8 within the cardiac pump housing 7. In the examples shown in figures 3 to 9 and 11, the first and second bearing portions 23a, 23b each comprise a substantially planar articular bearing surface arranged perpendicularly to the longitudinal axis A-A. In this manner, the first and second bearing portions 23a, 23b are configured to support the cardiac pump rotor 8 within the cardiac pump housing 7 in an axial direction of the cardiac pump rotor 8.

The first plain bearing portion 23a may comprise a spherical segment, i.e. a truncated spherical cap or spherical frustum. The second plain bearing portion 23b may be substantially disc-shaped. It is appreciated, however, that the first and second bearing portions 23a, 23b may be of any suitable form that permits the plain bearing assembly 23 to support the cardiac pump rotor 8 in at least the axial direction, for example, the first and/or second plain bearing portions 23a, 23b may comprise a frustoconical portion.

In an alternative example, the first and second bearing portions 23a, 23b may be arranged in any suitable manner such that the plain bearing assembly 23 is configured to support the cardiac pump rotor 8 within the cardiac pump housing 7 in at least a radial direction of the cardiac pump rotor 8. As such, the bearing surfaces of the first and second bearing portions 23a, 23b may be of any appropriate form. In one example, plain bearing assembly 23 may be configured to support the cardiac pump rotor 8 in the axial direction and in the radial direction, e.g. the first and second bearing portions 23a, 23b may comprise one or more curved, e.g. partially spherical, or conical bearing surfaces configured to be in rotatable contact. For example, the plain bearing assembly 23 may comprise an at least partial ball and socket bearing, wherein the one or more bearing surfaces of the first and second bearing portions 23a, 23b are substantially conformal. In general, the plain bearing assembly 23 may be configured such that the cardiac pump rotor 8 is substantially constrained in up to five degrees-of-freedom by any combination of point-, line- or surface-contact between the bearing surfaces of the first and second bearing portions 23a, 23b.

The area of contact between the first and second bearing portions 23a, 23b may be optimised with regard to heat generation and wear characteristics of the plain bearing assembly 23. For example, the area of contact may be a substantially circular contact area having an appropriate diameter that may be selected dependent upon operational characteristics of the cardiac pump 1 and the material from which the first and/or second bearing portions 23a, 23b are fabricated. In one example, the substantially circular contact area may have a diameter within a range of approximately 10 µm to 3 mm, or, in particular, within a range of approximately 300 µm to 1 mm. It is appreciated, however, that the shape of the contact area may be of any appropriate form and/or size. In another example, the plain bearing assembly 23 may comprise a plurality of contact areas, which may each be optimised to provide the desired levels of heat generation and wear characteristics.

In view of the above discussion, one important factor in the design of the cardiac pump 1 is the flow regime of the blood around the plain bearing assembly 23. For example, it is desirable to position the plain bearing assembly 23 in such a manner that it is exposed to a continuous flow of fresh blood for the purposes of washing the plain bearing assembly 23, for example the bearing interface of the plain bearing assembly 23, and disrupting any areas of flow stasis that may exist. For example, the non-rotating portion 23a of the plain bearing assembly 23 may be supported by a cage-like structure disposed within the inlet cannula 14 of the cardiac pump 1. In this manner, the bearing interface of the plain bearing assembly 23 is exposed to a high flow rate of blood, which serves to help wash the bearing interface and minimise the risk of protein deposition. However, since the plain bearing assembly 23 is provided at the radial centre of the cardiac pump, as it defines the rotational axis of the cardiac pump rotor 8, the plain bearing assembly 23 is exposed to a high axial flow of blood, and it is difficult to provide a substantial radial flow of fresh blood across the plain bearing interface.

The present disclosure is advantageous as it provides a cardiac pump 1 having a blood inlet 9 specially configured to direct blood radially across the plain bearing assembly 23 to ensure that the plain bearing assembly 23 is substantially washed with fresh blood in both an axial and a radial direction. The blood inlet 9 may, however, be configured to direct, for example redirect, blood towards or away from any appropriate feature of the cardiac pump.

Figures 2 to 5 show one arrangement of the cardiac pump 1 having an inlet 9 that is offset from the rotational axis A-A of the cardiac pump rotor 8, and hence from the radial location of the plain bearing assembly 23. For example, the inlet 9 comprises a single opening 27 having an axis B-B that is radially offset from the radial centre of the plain bearing assembly 23.

The opening 27 of figures 2 to 5 is shown as a circular opening provided parallel to a radial plane of the cardiac pump housing 7. However, the opening 27 may have any appropriate form and may be provided in any appropriate portion of the cardiac pump housing 7, such that the flow of blood through the inlet 9 of the inflow cannula 14 is radially offset from, e.g. eccentric from, the axis A-A.

For example, when the cardiac pump rotor 8 is supported within the cardiac pump housing 7, there is an annular gap between the radially outer surface of the cardiac pump rotor 8 and the radially inner surface of the cardiac pump housing 7. It is appreciated, therefore, that when the cardiac pump rotor 8 is mounted in the plain bearing assembly 23, the annular gap is substantially concentric with the axis A-A, and the pressure distribution within the annular gap is substantially uniform. As a result of the inlet 9 being radially offset from the axis A-A, the flow of blood that has entered the inlet 9 has a centre of pressure that is offset from the plain bearing assembly 23, which causes the blood flow to be directed radially across the plain bearing assembly 23. In this manner, the cardiac pump 1 according to the present disclosure provides a blood inlet 9 configured to impart a net radial component to the flow of blood entering the cardiac pump housing 7, which diverts blood flow radially across the bearing assembly 23. This is advantageous as the plain bearing assembly 23 is supplied with a continuous flow of fresh blood for the purposes of washing the bearing interface and disrupting any areas of flow stasis that may exist, therefore mitigating the risk of thrombus formation and/or the deposition of proteins in the region surrounding the plain bearing assembly 23.

In the arrangement of figures 2 to 5, the opening 27 is provided in an axial end of the cardiac pump 1 and extends axially though the cardiac pump housing 7, such that the blood flow though the opening 27 has an axial component parallel to the axis A-A of the cardiac pump 1. However, in one or more other arrangements, the opening 27 may extend though the cardiac pump housing 7 in any appropriate direction that results in a net radial blood flow across the bearing assembly 23. For example, the blood inlet 9 may comprise one or more further openings provided in the blood inflow cannula 14, the one or more opening being arranged to have a net centre of area that is offset from the axis A-A of the cardiac pump 1.

In the arrangement shown in figures 2 to 5, the opening 27 has a cross-sectional area, for example a reduced and/or minimum cross-sectional area, that is smaller than the cross-sectional area of the region of flow proximate to the blood inlet 9. Where the blood inlet comprises a plurality of openings, the total cross-sectional area of the plurality of openings may be smaller than the cross-sectional area of the region of flow proximate to the blood inlet 9. In this manner, for a given flow rate of blood through the cardiac pump 1, e.g. 3.5 litres per minute, the velocity of the blood flowing through the blood inlet 9 will be higher than the velocity of the blood flowing through the region of flow proximate to the blood inlet 9..

Figure 5 shows detail regarding the cross sectional areas of the inflow cannula 14 of the cardiac pump housing 7, and shows three representative cross sections of the flow path through the inflow cannula 14. For example, the blood inlet 9 may have a cross sectional area X through the blood inlet 9, a cross sectional area Y through a region of blood flow proximate to the blood inlet 9, for example a region of primary and/or secondary flow around the bearing assembly 23, and/or a cross sectional area Z through a region of flow in a space between the cardiac pump housing 7 and the cardiac pump rotor 8 when the cardiac pump 1 is in an assembled configuration, for example an annular blood gap between the cardiac pump housing 7 and the cardiac pump rotor 8. In the arrangement shown in figure 5, the blood inlet 9 is configured to flow blood into region of region of flow proximate to the blood inlet 9, past the bearing assembly 23, and subsequently into the annular blood gap.

In the arrangement of figure 5, the blood inlet 9 may have a minimum cross sectional area that defines the entry point into the inflow cannula 14. In this manner, it can be seen that the cross sectional area X of the blood inlet 9 may selected so as to vary the flow characteristics of the blood entering the inflow cannula 14. For example, the size of the cross sectional area X, or specifically the minimum cross sectional area, of the blood in let 9 may be selected to vary the velocity profile the blood entering the inflow cannula 14. As a result, the pressure profile in the blood, for example the pressure profile of the blood in a radial plane of the inflow cannula 14, may be resultant on the selected position and/or shape of the blood inlet 9.

In particular, the ratio of the cross sectional area X of the blood inlet 9 to the cross sectional area Y of the region of flow proximate to the blood inlet 9 may be selected to provide a desired pressure profile within the inflow cannula 14. In this manner, the present disclosure allows for the overall pressure distribution in the inflow cannula 14 to be selected so as to provide a substantial radial flow component in the blood that has passed through the blood inlet 9. Such a feature may be particularly advantageous as it allows for the flow regime in the pump to be designed so as to provide radial flow, e.g. cross-washing, of a component, such as a bearing assembly 23 of the cardiac pump 1. It is understood, therefore, that the pressure distribution may be defined by the position of the blood inlet 9 relative to the longitudinal axis A-A of the inflow cannula 14, in combination with the ratio of the cross sectional area X of the blood inlet 9 to the cross sectional area Y of the region of flow proximate to the blood inlet 9. In other words, the pressure distribution may be defined by the position of the cross sectional area, for example the minimum cross sectional area, of the blood inlet 9 relative to the longitudinal axis A-A of the inflow cannula 14, in combination with the internal geometry of the cardiac pump 1.

Additionally or alternatively, the ratio of the cross sectional area X of the blood inlet 9 to the cross sectional area Z of the annular blood gap may be selected to provide a desired pressure profile within the inflow cannula 14, in a similar manner.

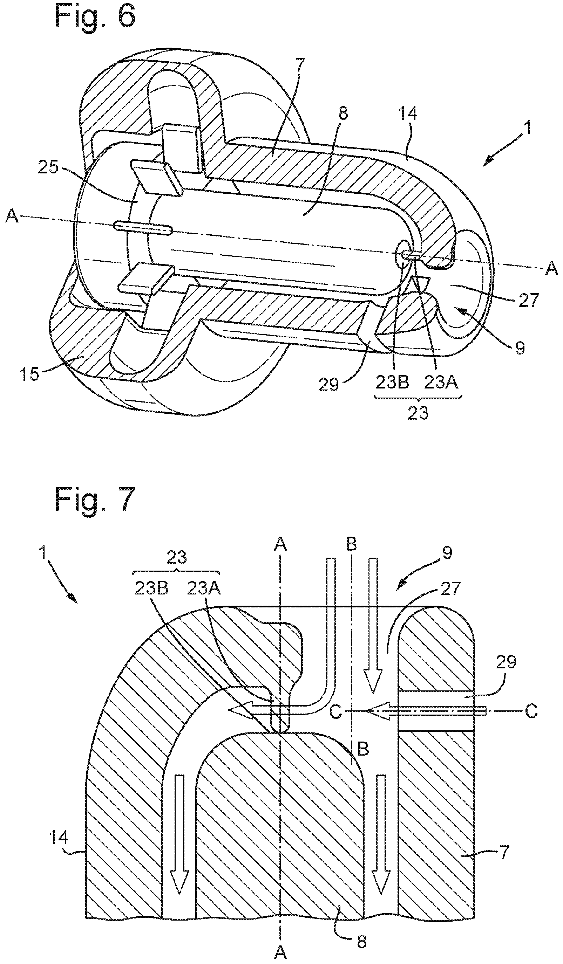

Figures 6 and 7 show another arrangement of the cardiac pump 1 that is provided with a blood inlet 9 having an axial opening 27 and a radial opening 29. In the arrangement of figures 6 and 7, the axial opening 27 comprises a circular opening similar to that of figures 2 to 5, and the radial opening 29 comprises an elongate slot extending radially through the inflow cannula 14 along an axis C-C. In the arrangement of figures 6 and 7, the axis B-B is perpendicular to the axis C-C. However, the axes B-B, C-C of the openings 27, 29 may extend in any appropriate direction. For example, the axes B-B, C-C of the openings 27, 29 may each be inclined to the longitudinal axis A-A of the inlet tube.

Where the blood inlet 9 comprises a plurality of openings 27, 29, the openings 27, 29 may be formed in the inflow cannula 14 such that the net flow of the blood into the inflow cannula 14 is radially offset from the longitudinal axis A-A of the inflow cannula 14. For example, if the plurality of openings 27, 29 are of the same size and shape, the plurality of openings 27, 29 may be positioned asymmetrically about the longitudinal axis A-A of the inflow cannula 14. In this manner, the pressure distribution of the blood flowing into the cardiac pump housing 7 will be radially offset from the longitudinal axis A-A, which will cause blood to flow radially across the plain bearing assembly 23. It can be seen, therefore, that where the blood inlet 9 has no rotational symmetry about the longitudinal axis A-A, e.g. a rotational symmetry of order 1 about the longitudinal axis A-A of the inflow cannula 14, an offset pressure distribution will be caused around the plain bearing assembly 23.

Where the plurality of openings 27, 29 are of different forms, they may be provided in a rotationally symmetric manner, since each of the plurality of openings27, 29 will provide a different flow rate into the cardiac pump housing 7, which serves to offset the pressure distribution around the plain bearing assembly 23 in a similar manner to that described above.

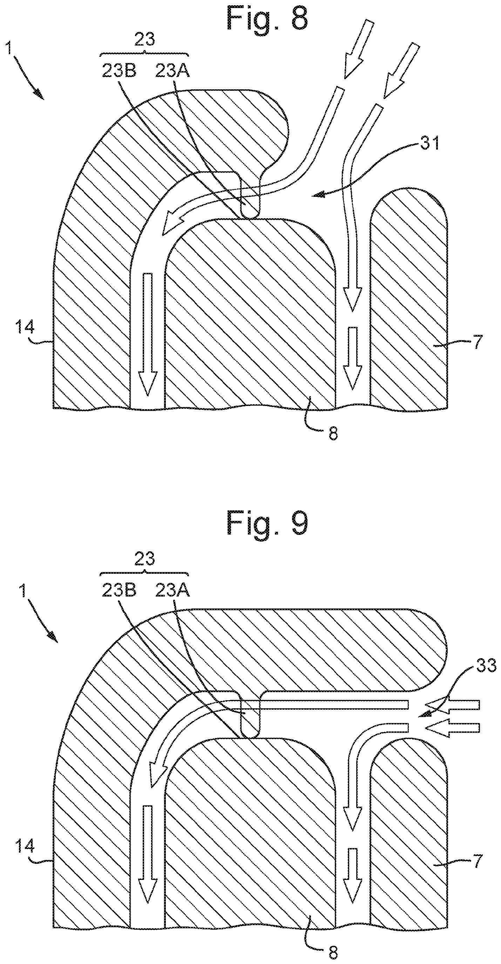

Figures 8 and 9 show other arrangements of the cardiac pump 1. In figure 8, the inflow cannula 14 comprises a blood inlet 9 formed from a single diagonal opening 31. The opening 31 allows blood to enter the inflow cannula 14 in both the axial and radial directions. In figure 9, the inflow cannula 14 comprises a blood inlet 9 formed from a single radial opening 33. The opening 33 allows blood to enter the inflow cannula 14 in a radial direction in a similar manner to the radial opening 29 shown in figures 5 and 6.

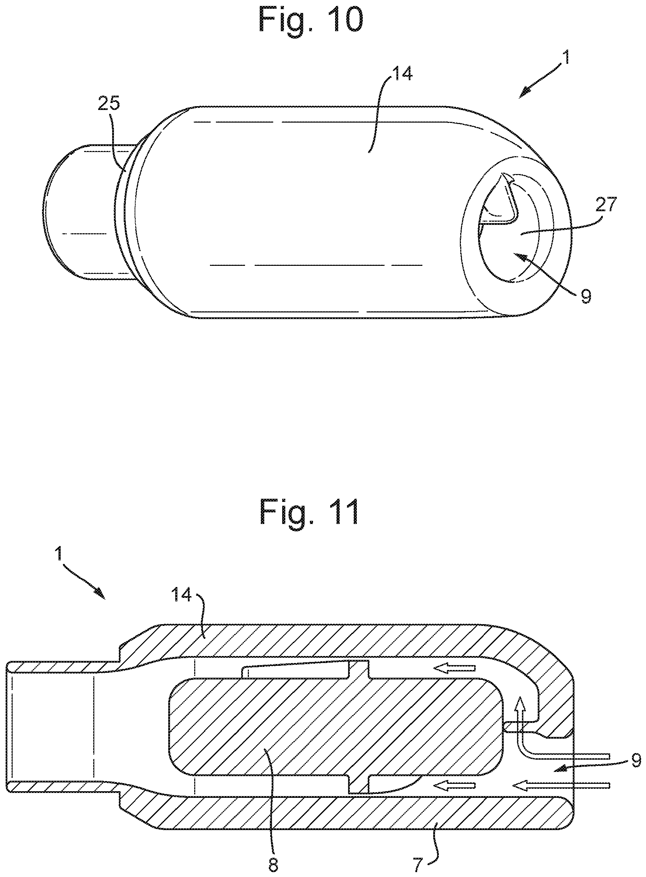

Figures 10 and 11 show another arrangement of the cardiac pump 1, in which the cardiac pump 1 comprises an axial flow cardiac pump. The axial flow cardiac pump comprises similar features to those shown in the radial flow arrangement of figures 1 to 9. The cardiac pump may be implanted in a similar manner to that shown in figure 1, or may be implanted in an alternate manner, for example outside of the heart or completely within the heart. It is appreciated, therefore, that the axial flow cardiac pump may function in substantially the same manner to supplement the function of the heart.

The axial flow cardiac pump comprises a cardiac pump housing 7 having a blood inlet 9 that is offset from the longitudinal axis A-A of the cardiac pump housing 7. In the arrangement of figures 10 and 11, the blood inlet 9 comprise a single opening 35 that is radially offset from the longitudinal axis A-A of the cardiac pump 1. The single opening 35 may, however, be configured in any appropriate manner according to the above description.

Irrespective of the configuration of the cardiac pump 1, it is understood that the blood inlet 9 may comprise any appropriate number of offset openings provided in an appropriate portion of the cardiac pump housing, such as the inflow cannula 14, in order to cause augmented radial flow in a region of the blood flow proximate to the blood inlet 9.

The present disclosure is advantageous, therefore, as it provides a cardiac pump housing having a blood inlet 9 that can be configured to improve the washing of a bearing assembly of a cardiac pump 1. In particular, the size, shape and/or position of the one or more offset openings 27, 29, 31, 33, 35 may be selected to provide a desired flow regime around a bearing assembly, such as a plain bearing assembly 23, of a cardiac pump 1. In this manner, the washing of the bearing assembly may be tuned depending on the desired use of the cardiac pump 1. For example, where the cardiac pump 1 is implanted into a first individual, it may be desirable to provide a first flow regime in the region surrounding the bearing assembly, and where the cardiac pump 1 is implanted into a second individual, it may be desirable to provide a second flow regime in the region surrounding the bearing assembly. The flow regime around the bearing assembly can therefore be selected depending on the condition of the individual and the physical characteristics of the individual's heart.

It will be appreciated by those skilled in the art that although the invention has been described by way of example with reference to one or more examples, it is not limited to the disclosed examples and that alternative examples could be constructed without departing from the scope of the invention as defined by the appended claims.

## Claims

1. A cardiac pump comprising:
a cardiac pump housing (7) having a blood inlet (9) that is offset from a longitudinal axis of the cardiac pump housing, the blood inlet being configured to flow blood radially across a contact bearing interface of a plain bearing assembly (23) of the cardiac pump.

2. A cardiac pump according to claim 1, wherein the blood inlet comprises one or more openings having a centre of area that is offset from the longitudinal axis of the cardiac pump housing.

3. A cardiac pump according to any of the preceding claims, wherein the blood inlet has a rotational symmetry of order 1 about the longitudinal axis of the cardiac pump housing.

4. A cardiac pump according to any of the preceding claims, wherein the blood inlet is non-axisymmetric.

5. A cardiac pump according to any of the preceding claims, the cardiac pump comprising at least one bearing assembly configured to rotatably support a cardiac pump rotor within the cardiac pump housing, wherein the blood inlet is configured to divert radially the flow of blood across the at least one bearing assembly.

6. A cardiac pump according to claim 5, wherein the at least one bearing assembly comprises a contact bearing having a first contact bearing portion configured to engage a second contact bearing portion thereby defining a contact bearing interface, the blood inlet being configured to divert radially the flow of blood across the contact bearing interface of the at least one bearing assembly.

7. A cardiac pump according to claim 6, wherein the blood inlet provides an opening into the blood flow path, the opening being disposed axially upstream of the contact bearing interface of the at least one bearing assembly.

8. A cardiac pump according to any preceding claim, wherein the cardiac pump housing defines a blood flow path between the blood inlet and a blood outlet of the cardiac pump housing, and wherein the blood flow path comprises a region of blood flow proximate to the blood inlet, wherein the cross sectional area of the flow through the blood inlet is a function of the cross sectional area of the region of blood flow proximate to the blood inlet.

9. A cardiac pump according to claim 7, wherein the ratio of the cross sectional area of the region of blood flow proximate to the blood inlet to the cross sectional area of the flow through the blood inlet is in the range of approximately 1:0.2 to 1:1.

10. A cardiac pump according to claim 7 or 8, wherein the cross sectional area of the region of blood flow proximate to the blood inlet is larger than the cross sectional area of the flow through the blood inlet.

11. A cardiac pump according to claim 5 or 6 and any of claims 7 to 9, wherein the at least one bearing assembly is positioned in the region of blood flow proximate to the blood inlet.

12. A cardiac pump according to any of the preceding claims, wherein the blood inlet forms a nozzle that leads into the cardiac pump housing.

13. A cardiac pump according to any of the preceding claims, wherein the blood inlet comprises one or more projections configured to offset radially the flow through the blood inlet.

14. A cardiac pump according to any of the preceding claims, wherein the blood inlet is configured to direct blood in a radial direction.

15. A cardiac pump according to any of the preceding claims, wherein the blood inlet is configured to establish a cross-flow of blood around, across and/or through another feature of the cardiac pump.

## Patentansprüche

1. Herzpumpe, umfassend:
ein Herzpumpengehäuse (7), das einen Bluteinlass (9) aufweist, der von einer Längsachse des Herzpumpengehäuses versetzt ist, wobei der Bluteinlass konfiguriert ist, um Blut radial über eine Kontaktlagerschnittfläche einer Gleitlageranordnung (23) der Herzpumpe zu leiten.

2. Herzpumpe gemäß Anspruch 1, wobei der Blutzufluss eine oder mehrere Öffnungen aufweist, die einen Flächenschwerpunkt aufweisen, der gegenüber der Längsachse des Herzpumpengehäuses versetzt ist.

3. Herzpumpe gemäß einem der vorherigen Ansprüche, wobei der Bluteinlass eine Rotationssymmetrie der Ordnung 1 um die Längsachse des Herzpumpengehäuses aufweist.

4. Herzpumpe gemäß einem der vorherigen Ansprüche, wobei der Bluteinlass nicht achsensymmetrisch ist.

5. Herzpumpe gemäß einem der vorherigen Ansprüche, die Herzpumpe umfassend mindestens eine Lageranordnung, die konfiguriert ist, um einen Herzpumpenrotor innerhalb des Herzpumpengehäuses drehbar zu lagern, wobei der Bluteinlass konfiguriert ist, um den Blutstrom radial über die mindestens eine Lageranordnung umzuleiten.

6. Herzpumpe gemäß Anspruch 5, wobei die mindestens eine Lageranordnung ein Kontaktlager umfasst, das einen ersten Kontaktlagerabschnitt aufweist, der konfiguriert ist, um einen zweiten Kontaktlagerabschnitt einzugreifen, wodurch eine Kontaktlagergrenzfläche definiert ist, wobei der Bluteinlass konfiguriert ist, um den Blutstrom radial über die Kontaktlagerschnittfläche der mindestens einen Lageranordnung umzuleiten.

7. Herzpumpe gemäß Anspruch 6, wobei der Bluteinlass eine Öffnung in den Blutströmungsweg bereitstellt, wobei die Öffnung axial stromaufwärts von der Kontaktlagerschnittfläche der mindestens einen Lageranordnung angeordnet ist.

8. Herzpumpe gemäß einem der vorherigen Ansprüche, wobei das Herzpumpengehäuse einen Blutströmungsweg zwischen dem Bluteinlass und einem Blutauslass des Herzpumpengehäuses definiert, und wobei der Blutströmungsweg eine Region von Blutströmung in der Nähe des Bluteinlasses umfasst, wobei die Querschnittsfläche der Strömung durch den Bluteinlass abhängig von der Querschnittsfläche der Region von Blutströmung in der Nähe des Bluteinlasses ist.

9. Herzpumpe gemäß Anspruch 7, wobei das Verhältnis der Querschnittsfläche der Region von Blutströmung in der Nähe des Bluteinlasses zu der Querschnittsfläche der Strömung durch den Bluteinlass in dem Bereich von etwa 1:0,2 bis 1:1 ist.

10. Herzpumpe gemäß Anspruch 7 oder 8, wobei die Querschnittsfläche der Region von Blutströmung in der Nähe des Bluteinlasses größer ist als die Querschnittsfläche der Strömung durch den Bluteinlass.

11. Herzpumpe gemäß Anspruch 5 oder 6 und einem der Ansprüche 7 bis 9, wobei die mindestens eine Lageranordnung in der Region der Blutströmung in der Nähe des Bluteinlasses angeordnet ist.

12. Herzpumpe gemäß einem der vorherigen Ansprüche, wobei der Bluteinlass eine Düse bildet, die in das Herzpumpengehäuse führt.

13. Herzpumpe gemäß einem der vorherigen Ansprüche, wobei der Bluteinlass einen oder mehrere Vorsprünge umfasst, die konfiguriert sind, um die Strömung durch den Bluteinlass radial zu versetzen.

14. Herzpumpe gemäß einem der vorherigen Ansprüche, wobei der Bluteinlass konfiguriert ist, um Blut in eine radiale Richtung zu richten.

15. Herzpumpe gemäß einem der vorherigen Ansprüche, wobei der Bluteinlass konfiguriert ist, um eine Querströmung von Blut um, über und/oder durch ein anderes Merkmal der Herzpumpe herzustellen.

## Revendications

1. Une pompe cardiaque comprenant :
un boîtier de pompe cardiaque (7) ayant une entrée de sang (9) qui est décalée à partir d'un axe longitudinal du boîtier de pompe cardiaque, l'entrée de sang étant configurée pour faire circuler le sang radialement à travers une interface de palier de contact d'un ensemble palier lisse (23) de la pompe cardiaque.

2. Une pompe cardiaque selon la revendication 1, dans laquelle l'entrée de sang comprend une ou plusieurs ouvertures ayant un centre de zone qui est décalé à partir de l'axe longitudinal du boîtier de pompe cardiaque.

3. Une pompe cardiaque selon l'une quelconque des revendications précédentes, dans laquelle l'entrée de sang a une symétrie de rotation d'ordre 1 autour de l'axe longitudinal du boîtier de pompe cardiaque.

4. Une pompe cardiaque selon l'une quelconque des revendications précédentes, dans laquelle l'entrée de sang est non axisymétrique.

5. Une pompe cardiaque selon l'une quelconque des revendications précédentes, la pompe cardiaque comprenant au moins un ensemble palier configuré pour supporter de manière rotative un rotor de pompe cardiaque à l'intérieur du boîtier de pompe cardiaque, dans laquelle l'entrée de sang est configurée pour dévier radialement l'écoulement du sang à travers l'au moins un ensemble palier.

6. Une pompe cardiaque selon la revendication 5, dans laquelle l'au moins un ensemble palier comprend un palier de contact ayant une première partie de palier de contact configurée pour engager une deuxième partie de palier de contact définissant ainsi une interface de palier de contact, l'entrée de sang étant configurée pour dévier radialement l'écoulement du sang à travers l'interface de palier de contact de l'au moins un ensemble palier.

7. Une pompe cardiaque selon la revendication 6, dans laquelle l'entrée de sang fournit une ouverture dans le trajet d'écoulement du sang, l'ouverture étant disposée axialement en amont de l'interface de palier de contact de l'au moins un ensemble palier.

8. Une pompe cardiaque selon l'une quelconque des revendications précédentes, dans laquelle le boîtier de pompe cardiaque définit un trajet d'écoulement du sang entre l'entrée de sang et une sortie de sang du boîtier de pompe cardiaque, et dans laquelle le trajet d'écoulement du sang comprend une région d'écoulement du sang à proximité de l'entrée de sang, dans laquelle la section transversale de l'écoulement à travers l'entrée de sang est une fonction de la section transversale de la région de l'écoulement du sang à proximité de l'entrée de sang.

9. Une pompe cardiaque selon la revendication 7, dans laquelle le rapport de la section transversale de la région de l'écoulement du sang à proximité de l'entrée de sang à la section transversale de l'écoulement à travers l'entrée de sang est dans la plage d'environ 1:0,2 à 1:1.

10. Une pompe cardiaque selon la revendication 7 ou 8, dans laquelle la section transversale de la région de l'écoulement du sang à proximité de l'entrée de sang est plus grande que la section transversale de l'écoulement à travers l'entrée de sang.

11. Une pompe cardiaque selon la revendication 5 ou 6 et l'une quelconque des revendications 7 à 9, dans laquelle l'au moins un ensemble palier est positionné dans la région de l'écoulement du sang à proximité de l'entrée de sang.

12. Une pompe cardiaque selon l'une quelconque des revendications précédentes, dans laquelle l'entrée de sang forme une buse qui mène dans le boîtier de pompe cardiaque.

13. Une pompe cardiaque selon l'une quelconque des revendications précédentes, dans laquelle l'entrée de sang comprend une ou plusieurs saillies configurées pour décaler radialement l'écoulement à travers l'entrée de sang.

14. Une pompe cardiaque selon l'une quelconque des revendications précédentes, dans laquelle l'entrée de sang est configurée pour diriger le sang dans une direction radiale.

15. Une pompe cardiaque selon l'une quelconque des revendications précédentes, dans laquelle l'entrée de sang est configurée pour établir un écoulement transversal du sang autour, au-delà et / ou à travers une autre caractéristique de la pompe cardiaque.
